# EUROPEAN PATENT APPLICATION

(11) **EP 4 792 845 A1**
(43) Date of publication of application: **19.08.2026**
(21) Application number: 25157603.9
(22) Date of filing: 13.02.2025
(51) Int. Cl.: A61M 1/06

(54) **MILK EXPRESSION KIT AND BREAST PUMP ASSEMBLY**

(71) Applicant: Koninklijke Philips N.V., 5656 AE Eindhoven (NL)
(72) Inventor: LUI, Kwan Fai, Eindhoven (NL); DOBRUSSKIN, Christoph, Eindhoven (NL); CARTEI, Mirko, Eindhoven (NL); JIANG, Yong, Eindhoven (NL)
(74) Representative: Philips Intellectual Property & Standards

(57) **Abstract**

Provided is a milk expression kit (10) that has a nipple-receiving member (52) in which a mother's nipple (NP) is at least partially receivable, a diaphragm holder (102), and a breast pump diaphragm (100) for mounting to the diaphragm holder. The diaphragm includes a first exterior surface (104), with a pump chamber being defined between the diaphragm holder and the first exterior surface when the milk expression kit is assembled for use. The diaphragm also includes a second exterior surface (106) facing away from the first exterior surface. The diaphragm is configured to transfer a variable pressure provided in the pump chamber to a breast chamber that is at least partly delimited by the second exterior surface. Further provided is a breast pump assembly comprising a variable pressure generator and the milk expression kit.

## Description

### FIELD OF THE INVENTION

The invention relates to a milk expression kit comprising a breast pump diaphragm. The invention further relates to a breast pump assembly comprising the milk expression kit.

### BACKGROUND OF THE INVENTION

Breast pump assemblies are widely used by lactating mothers to express milk. Reasons for using such breast pump assemblies for expressing milk include feeding convenience, milk supply regulation, and relieving breast engorgement. The effectiveness of the breast pump assembly may depend on various factors, including capability to create a proper seal around the nipple and generate sufficient vacuum pressure for milk extraction.

Design of the breast pump assembly's diaphragm may be key to generating sufficient vacuum pressure. The diaphragm may move/flex in order to transfer a variable pressure from a pump chamber, that is at least partly delimited by the diaphragm, to a breast chamber, which breast chamber is fluidly connected to the mother's breast.

Fit of the diaphragm in the breast pump assembly, for example in a milk expression kit thereof, can, however, be sub-optimal. This can result in reduced milk flow/inefficient milk extraction, and/or can cause discomfort to the mother during milk extraction, for example due to her nipple being unintentionally contacted by the moving/flexing diaphragm.

### SUMMARY OF THE INVENTION

The invention is defined by the claims.

According to examples in accordance with a first aspect of the present disclosure, there is provided a milk expression kit comprising: a nipple-receiving member in which a mother's nipple is at least partially receivable; a diaphragm holder; a breast pump diaphragm for mounting to the diaphragm holder, an extremity of the nipple-receiving member being located at a position inside the milk expression kit proximal to the diaphragm, with a diaphragm movement region being defined between the extremity and one of (i) the diaphragm and (ii) the diaphragm holder (102), when the milk expression kit is assembled for use, the diaphragm comprising: a first exterior surface, a pump chamber being defined between the diaphragm holder and the first exterior surface when the milk expression kit is assembled for use; a second exterior surface facing away from the first exterior surface, the diaphragm being configured to transfer a variable pressure provided in the pump chamber to a breast chamber that is at least partly delimited by the second exterior surface; wherein a recessed portion of the second exterior surface is defined in a central region of the diaphragm; a peripheral portion having a polygonal shape in plan when the diaphragm is viewed facing the first exterior surface or the second exterior surface; and an intermediate portion located between the recessed portion and at least vertex regions of the peripheral portion's polygonal shape.

The diaphragm can be mounted to the diaphragm holder so that the first exterior surface of the diaphragm faces the diaphragm holder.

Alternatively or additionally, the diaphragm can be mounted to the diaphragm holder so that the second exterior surface faces away from the diaphragm holder, e.g. towards a nipple-receiving space defined in the milk expression kit when assembled for use.

The diaphragm movement region defined between one of (i) the diaphragm and (ii) the diaphragm holder (102), can be regarded as a region in which movement and/or flexing of the diaphragm is permitted.

It is noted that the diaphragm movement region can still allow movement/flexing of the diaphragm therein despite the diaphragm having, for example, a crease or other protruding region that touches the extremity but still allows the movement/flexing of the diaphragm to create the variable pressure, e.g. variable under-pressure.

Alternatively, the extremity may have a protruding feature that makes contact with the diaphragm but still allows the movement/flexing of the diaphragm to create the variable pressure, e.g. variable under-pressure.

In some embodiments, the milk expression kit is configured so that, when assembled for use, positioning of the nipple-receiving member relative to the diaphragm is such that the nipple-receiving member and the diaphragm are spatially separated from, i.e. not in physical contact with, each other.

This may assist movement/flexing of the diaphragm relative to the nipple-receiving member.

In some embodiments, the extremity of the nipple-receiving member is located at a position inside the milk expression device proximal to, but without touching, the diaphragm when the milk expression kit is assembled for use.

In such embodiments, the diaphragm may be allowed to move/flex without interference that can otherwise result from physical contact between the extremity of the nipple-receiving member and the diaphragm.

The diaphragm may move relative to, but without touching, the extremity of the nipple-receiving member throughout provision of the variable pressure in the pump chamber by a variable pressure generator of a breast pump assembly in which the milk expression kit is also included.

The recessed portion defined in the second exterior surface of the diaphragm can help to make the milk expression kit more compact, e.g. so as to facilitate fitting of the milk expression kit in the mother's bra. For example, the recessed portion can enable the diaphragm to be arranged closer to the breast, with less risk of the nipple contacting the diaphragm due to the recessed region of the second exterior surface providing additional space between the nipple and the diaphragm.

It is noted that the recessed region of the second exterior surface may correspond to a protruding region of the first exterior surface.

For example, a profile of the first exterior surface may complement a profile of the second exterior surface, such that recessed region(s) of one of the first and second exterior surfaces correspond to protruding region(s) of the other of the first and second exterior surfaces.

The polygonal peripheral portion together with the intermediate portion of the diaphragm, located between the recessed portion and at least vertex regions of the peripheral portion's polygonal shape, can assist to increase the surface area of the diaphragm, in other words can provide an additional surface of the diaphragm that can be moved, e.g. stretched, for vacuum generation.

In some embodiments, the diaphragm holder comprises a polygonal mounting portion, with the polygonal shape of the diaphragm's peripheral portion enabling the diaphragm to be fitted to the polygonal mounting portion of the diaphragm holder. The polygonal shaped peripheral portion can indicate that the diaphragm is correctly mounted to the diaphragm holder, due to the complementary fit of the polygonal shaped peripheral portion and the polygonal mounting portion of the diaphragm holder to each other.

The polygonal shaped peripheral portion in combination with the polygonal mounting portion of the diaphragm holder may also assist to ensure secure, non-rotatable mounting of the diaphragm in the diaphragm holder.

In some embodiments, the diaphragm has a three-dimensional shape having an axis of rotational symmetry that extends through a center of the polygonal shape to enable the diaphragm to be fitted to the polygonal mounting portion in a plurality of angular orientations adoptable by rotating the diaphragm about the axis. This can assist to enhance user convenience, since the user is not burdened with having to locate, for example, a single orientation of the diaphragm that enables the diaphragm to be mounted to the diaphragm holder.

The center of the polygonal shape can be regarded as being defined by the axis of rotational symmetry, with rotation about the axis providing the plurality of (discrete) angular orientations.

It is noted that the angular orientations may be regarded as being indistinguishable from each other in terms of functioning of the diaphragm in the assembled milk expression kit.

In some embodiments, the three-dimensional shape of the diaphragm has an order of rotational symmetry, about the axis of rotational symmetry, of between 2 and 10, preferably between 3 and 6, more preferably between 4 and 6. For example, in the case of the polygonal shape being a square or rounded square shape, the order of rotational symmetry is 4. In such embodiments, the diaphragm may be mounted to the diaphragm holder in one of four angular orientations.

In other embodiments, in which the polygonal shape is an oblong shape, e.g. rectangular, so as to have a length dimension longer than a width dimension, the order of rotational symmetry is 2. In such embodiments, the diaphragm may be mounted to the diaphragm holder in one of two angular orientations.

In some embodiments, a depth of the recessed portion is at least one third of a width of the recessed portion, with the depth being measured along the axis of rotational symmetry and the width being measured across the recessed region perpendicular to the axis of rotational symmetry. This minimum depth requirement reflects statistical data gathered concerning nipple length to width ratios. In particular, the depth of the recessed portion being at least one third of the width of the recessed portion may enable the breast pump assembly, or at least the milk expression kit thereof, to accommodate a largest nipple length to width ratio.

In some embodiments, the recessed portion has a circular shape in plan when the diaphragm is viewed facing the second exterior surface. For example, the recessed portion may be dome-shaped.

Such a dome-shaped recessed portion can assist to ensure that the diaphragm is always able to return to its original shape during pumping, which can help to create a stronger vacuum force. The stronger vacuum force can, in turn, help to enhance efficiency of milk extraction.

Alternatively, the recessed portion may be cylindrical.

It is noted that the shapes, e.g. circular shape in plan, dome-shaped and cylindrical, mentioned herein in relation to the recessed portion may refer to the shapes when the diaphragm is not being deformed by a pressure difference between the first and second exterior surfaces of the diaphragm.

In some embodiments, ridge(s) and/or groove(s) is/are defined in at least the second exterior surface of the diaphragm. Such ridge(s) and/or groove(s) can help to provide smooth milk flow off the second exterior surface of the diaphragm, thereby ensuring efficient milk collection.

In some embodiments, a groove, e.g. an annular groove, defined in the second exterior surface corresponds to a ridge, e.g. an annular ridge, of the first exterior surface.

Alternatively or additionally, a ridge, e.g. an annular ridge, of the second exterior surface corresponds to a groove, e.g. an annular groove, defined in the first exterior surface.

In embodiments in which the recessed portion of the second exterior surface is defined in the central region of the diaphragm, the ridge(s) and/or groove(s) may extend at least partly, e.g. entirely, around the recessed region. For example, the ridge(s) and/or groove(s) may annularly extend around the recessed region, e.g. the dome-shaped recessed region.

The ridge(s) and/or groove(s) extending, e.g. annularly extending, around the recessed region can help to increase the distance to which the diaphragm is able to extend, thereby assisting to create a relatively high vacuum force.

As an alternative or in addition to the ridge(s) and/or groove(s) extending at least partly around the recessed region, the ridge(s) and/or groove(s) may be defined on/in the recessed region itself and/or on/in the protruding region of the first exterior surface that corresponds to the recessed region of the second exterior surface.

The diaphragm may be formed of any suitable material. In some embodiments, the diaphragm comprises an elastomeric material. Thus, the diaphragm may be resiliently deformable. In some embodiments, the elastomeric material is silicone.

In at least some embodiments, the milk expression kit comprises a milk collection container.

The milk collection container may, for example, be wearable inside the mother's bra together with the diaphragm and the diaphragm holder.

In some embodiments, the diaphragm is arranged in the milk expression kit such that at least part of the second exterior surface is, in use, angled to guide milk thereon towards the milk collection container. Thus, the orientation of at least part of the second exterior surface of the diaphragm can assist transfer of milk towards the milk collection container.

In some embodiments, the diaphragm as a whole may be angled so that an overall orientation of the second exterior surface, in use, guides milk thereon towards the milk collection container.

When the milk expression kit is assembled, the diaphragm may be tilted such that the axis of rotational symmetry extends at an oblique angle relative to a central axis that extends through a center of the nipple-receiving space towards the diaphragm.

This tilting of the diaphragm may cause milk to be guided on the second exterior surface towards the milk collection container.

In other embodiments, a part of the second exterior surface may be angled relative to neighboring part(s) of the second exterior surface, so that the part of the second exterior surface guides milk thereon towards the milk collection container.

In some embodiments, the positioning of the nipple-receiving member relative to the diaphragm, in the milk expression kit assembled for use, may be such that the nipple-receiving space inside the nipple-receiving member is aligned with the recessed portion of the second exterior surface. In such embodiments, the recessed portion can enable the diaphragm to be arranged closer to the breast, with less risk of the nipple contacting the diaphragm due to the recessed region of the second exterior surface providing additional space between the nipple and the diaphragm.

By arranging the diaphragm closer to the breast, the overall thickness of the milk expression kit can be reduced, so as to take up less room in the mother's bra.

The alignment between the nipple-receiving space and the recessed portion can mean that the recessed portion and the nipple-receiving space are opposite each other, for example so that a central axis extending through a center of the nipple-receiving space intersects with the diaphragm at the recessed portion.

It is noted that the intersection of the central axis extending through the center of the nipple-receiving space with the diaphragm at the recessed portion need not require that the center of the diaphragm is intersected by the central axis. For example, in embodiments in which the diaphragm is angled so that an overall orientation of the second exterior surface, in use, guides milk thereon towards the milk collection container, the central axis extending through the center of the nipple-receiving space may intersect the diaphragm at an off-center position due, at least in part, to the tilted orientation of the diaphragm.

More generally, the milk expression kit may be an in-bra wearable milk expression kit.

In other words, the milk expression kit may be dimensioned and shaped so as to be wearable within the mother's bra.

In some embodiments, at least the diaphragm holder, the diaphragm, and the nipple-receiving member of the milk expression kit are received in the mother's bra when the milk expression kit is being worn by the mother.

According to examples in accordance with a second aspect of the present disclosure, there is provided a breast pump assembly comprising: a variable pressure generator; and the milk expression kit according to any of the embodiments described herein, wherein the variable pressure generator is configured to generate a variable pressure between the first exterior surface of the diaphragm and the diaphragm holder.

When the variable pressure generator, e.g. pump, is providing the variable pressure between the first exterior surface of the diaphragm and the diaphragm holder, at least a central part of the first exterior surface of the diaphragm (e.g. at least the protruding region corresponding to the recessed region of the second exterior surface) may move towards and away from the diaphragm holder. For example, the central part of the first exterior surface, e.g. the protruding region thereof, may move towards and touch an opposing surface of the diaphragm holder when the variable pressure is being provided.

In other embodiments, the diaphragm may be configured so that a central part, e.g. the protruding region, of the first exterior surface of the diaphragm may not move towards the diaphragm holder in response to the variable pressure being provided, but rather surrounding part(s) of the diaphragm may move towards and away from the diaphragm holder while the central part remains stationary relative to the diaphragm holder.

This may mean that even less space is used, so that the breast pump assembly, or at least the milk expression kit thereof, can be made more compact.

In some embodiments, the variable pressure generator is wearable and configured to generate the variable pressure while being worn. For example, the variable pressure generator may be wearable inside the mother's bra. Alternatively, the variable pressure generator may be wearable by the mother, but outside the mother's bra.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 provides a cutaway view of a milk expression kit according to an example;
FIG. 2A provides a cutaway view of a breast pump diaphragm included in the milk expression kit shown in FIG. 1;
FIG. 2B provides a plan view of the diaphragm shown in FIG. 2A; and
FIG. 2C provides a perspective view of the diaphragm shown in FIGs. 2A and 2B.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a milk expression kit that has a nipple-receiving member in which a mother's nipple is at least partially receivable, a diaphragm holder, and a breast pump diaphragm for mounting to the diaphragm holder. The diaphragm includes a first exterior surface, with a pump chamber being defined between the diaphragm holder and the first exterior surface when the milk expression kit is assembled for use. The diaphragm also includes a second exterior surface facing away from the first exterior surface. The diaphragm is configured to transfer a variable pressure provided in the pump chamber to a breast chamber that is at least partly delimited by the second exterior surface.

Further provided is a breast pump assembly comprising a variable pressure generator and the milk expression kit.

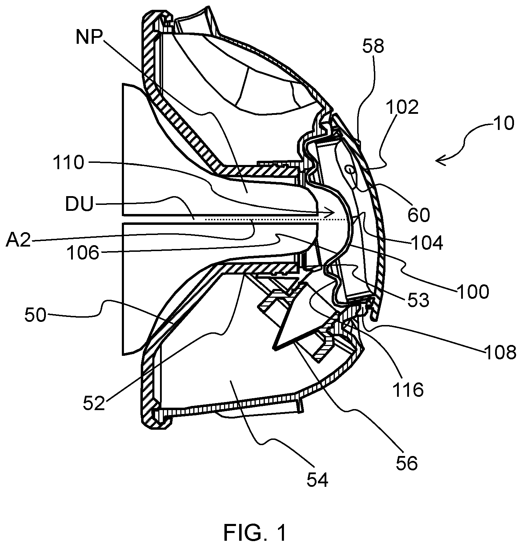

FIG. 1 shows a milk expression kit 10 according to an example. The milk expression kit 10 may be an in-bra wearable milk expression kit 10 that is wearable by a mother by inserting the milk expression kit 10 into her bra.

The milk expression kit 10 may include a breast shield 50, also known as a breast funnel, for interfacing with the mother's breast.

The breast shield 50 can be formed from any suitable material. In some embodiments, the breast shield 50 is formed from a plastic material, e.g. polypropylene, and/or an elastomeric material, e.g. silicone rubber.

It is noted that the resilience of the elastomeric material, when the elastomeric material is included in, e.g. forms, the breast shield 50, may assist to make the breast shield 50 more comfortable for the mother to wear on her breast.

The milk expression kit 10 comprises a nipple-receiving member 52 in which the mother's nipple NP is at least partially receivable. The nipple-receiving member 52 may, for example, form part of the breast shield 50, as in the non-limiting example shown in FIG. 1. Alternatively, the nipple-receiving member 52 may be provided as a separate piece with respect to the breast shield 50. Such a separate piece may, for example, be detachable from the breast shield 50.

During interfacing with the mother's breast, the nipple NP may be moved into the nipple-receiving member 52 in the direction of an extremity 53 of the nipple-receiving member 52.

The nipple-receiving member 52 can, similarly to the breast shield 50, be formed from any suitable material. In some embodiments, the nipple-receiving member 52 is formed from a plastic material, e.g. polypropylene, and/or an elastomeric material, e.g. silicone rubber.

The milk expression kit 10 may comprise a milk collection container 54. In some embodiments, such as shown in FIG. 1, the milk collection container 54 is wearable, together with the rest of the milk expression kit 10, inside the mother's bra.

The milk collection container 54 can be formed, e.g. molded, from any suitable material, such as a plastic material.

The milk collection container 54 may, for example, be at least partly delimited by an optically transmissive wall, for instance made of an optically transmissive plastic material, that allows visual inspection of how much milk has been received in the milk collection container 54.

In some embodiments, the milk collection container 54 is equipped with a milk level indicator for providing the mother with a visual indication of how much milk has been received in the milk collection container 54.

The milk level indicator can take any suitable form. In some embodiments, the milk level indicator comprises measurement markings and/or surface discontinuity(ies), such as ledge(s), interrupting uniform extension of a surface of a wall delimiting the milk collection container 54.

The milk level indicator, e.g. the surface discontinuity(ies) and/or measurement markings, may be arranged so as to be visible to the mother while she is wearing the milk collection container 54.

In this way, the visual indication of how much milk has been received in the milk collection container 54 may be provided to the mother while she is expressing milk into the milk collection container 54.

The milk collection container 54 may be arranged beneath the nipple-receiving member 52 when the milk expression kit 10 is orientated for use, so that collection of milk in the milk collection container 54 is assisted by gravity.

The milk expression kit 10 may include a one-way valve 56, such as a duckbill valve, configured to permit milk to flow therethrough into the milk collection container 54, but restrict backflow of milk away from the milk collection container 54 towards the nipple NP.

A variable pressure generator, e.g. pump, may be connected to the milk expression kit 10 to provide a variable pressure, e.g. a varying under-pressure, for assisting extraction of milk from the nipple NP.

The variable pressure may assist extraction of milk from milk ducts DU in the nipple NP, with the extracted milk being received in the milk expression kit 10, e.g. in the milk collection container 54 thereof.

In some embodiments, the variable pressure generator is wearable and configured to generate the variable pressure while being worn. For example, the variable pressure generator may be wearable inside the mother's bra. Alternatively, the variable pressure generator may be wearable by the mother, but outside the mother's bra.

In some embodiments, such as shown in FIG. 1, the milk expression kit 10 comprises a connector 58 for connecting the milk expression kit 10 to the variable pressure generator, for instance for connecting the milk expression kit 10 to a tube, which tube fluidly connects the milk expression kit 10 to the variable pressure generator.

Such a tube may, for instance, fluidly connect the milk expression kit 10 to the variable pressure generator worn by the mother, but outside the mother's bra.

A channel 60 may be defined through part of the milk expression kit's 10 casing, which channel 60 provides fluid communication between the variable pressure generator and an inside of the milk expression kit that is at least partly delimited by the casing.

The present disclosure relates, in part, to the milk expression kit 10 as such, since the milk expression kit 10 can be supplied to the mother by itself, with the variable pressure generator being already in the mother's possession, or sourced separately from the milk expression kit 10.

Alternatively, the milk expression kit 10 and the variable pressure generator may both be included in a breast pump assembly. In such embodiments, the mother receiving the breast pump assembly may be conveniently supplied with the milk expression kit 10 together with the variable pressure generator.

With continued reference to FIG. 1, the milk expression kit 10 comprises a breast pump diaphragm 100 and a diaphragm holder 102 to which the diaphragm 100 is mountable. The diaphragm 100 has a first exterior surface 104 and a second exterior surface 106.

The first exterior surface 104 may face the diaphragm holder 102 when the milk expression kit 10 is assembled for use with the diaphragm 100 mounted to the diaphragm holder 102, with the second exterior surface 106 facing away from the diaphragm holder 102, e.g. towards the mother's breast.

A pump chamber is defined between the first exterior surface 104 of the diaphragm 100 and the diaphragm holder 102, and a breast chamber is at least partly delimited by the second exterior surface 106 of the diaphragm 100.

The diaphragm 100 may move/flex in order to transfer the variable pressure from the pump chamber to the breast chamber, with the variable pressure provided in the pump chamber being generated by the variable pressure generator that is fluidly connected to the pump chamber, e.g. via the connector 58 and/or the channel 60.

In some embodiments, such as shown in FIG. 1, the channel 60 extends through the diaphragm holder 102, to enable the fluid connection to be provided between the variable pressure generator, e.g. pump, and the pump chamber.

It is generally noted that the diaphragm 100 may separate the pump chamber and the breast chamber from each other, so that the diaphragm 100 prevents milk in the breast chamber from reaching the variable pressure generator, e.g. pump, that is in fluid communication with the pump chamber.

This barrier function of the diaphragm 100 can be regarded as providing a closed system-type milk expression kit 10 or breast pump assembly.

The diaphragm 100 may be formed of any suitable material. In some embodiments, the diaphragm 100 comprises an elastomeric material, such as silicone. Thus, the diaphragm 100 may be resiliently deformable.

The milk expression kit 10 shown in FIG. 1 is configured so that a positioning of the nipple-receiving member 52 relative to the diaphragm 100, when the milk expression kit 10 is assembled for use, is such that the nipple-receiving member 52 and the diaphragm 100 are spatially separated from, i.e. not in physical contact with, each other. This may assist movement/flexing of the diaphragm 100 relative to the nipple-receiving member 52.

As shown in FIG. 1, the extremity 53 of the nipple-receiving member 52 is located inside the milk expression kit 10 in a position proximal to, but without touching, the diaphragm 100 when the milk expression kit 10 is assembled for use. Thus, the diaphragm 100 may be allowed to move/flex without interference that can otherwise result from physical contact between the extremity 53 of the nipple-receiving member 52 and the diaphragm 100.

In more general terms, a diaphragm movement region can be defined between the diaphragm 100 and the extremity 53 of the nipple-receiving member 52.

The diaphragm movement region can be regarded as a region in which movement and/or flexing of the diaphragm 100 is permitted.

It is noted that the diaphragm movement region can still allow movement/flexing of the diaphragm 100 therein despite the diaphragm 100 having, for example, a crease or other protruding region that touches the extremity 53 but still allows the movement/flexing of the diaphragm 100 to create the variable pressure, e.g. variable under-pressure.

Alternatively, the extremity 53 may have a protruding feature that makes contact with the diaphragm 100 but still allows the movement/flexing of the diaphragm 100 to create the variable pressure, e.g. variable under-pressure.

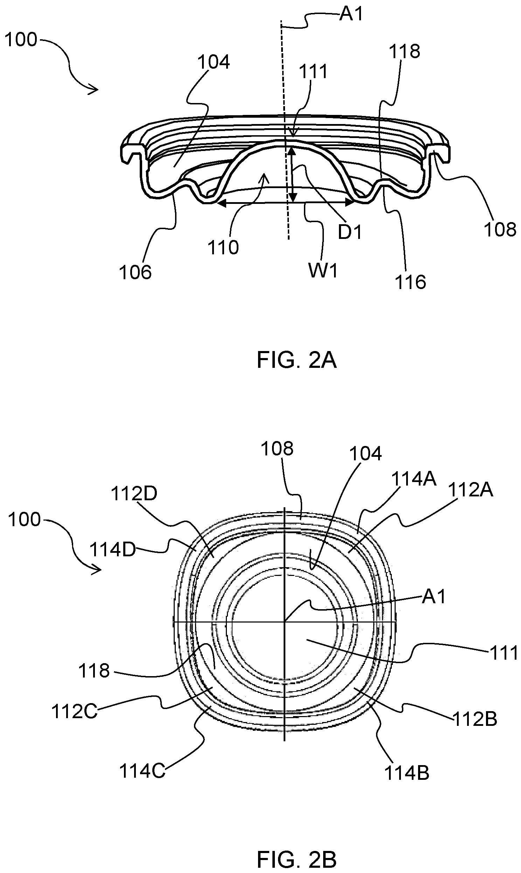

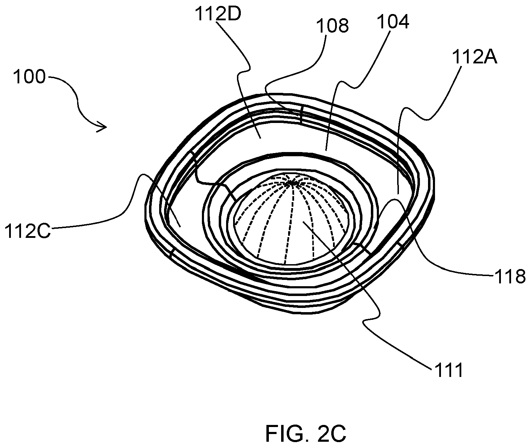

Referring now to FIGs. 2A to 2C, the diaphragm 100 comprises a peripheral portion 108 that has a polygonal shape in plan when the diaphragm 100 is viewed facing the first exterior surface 104 or the second exterior surface 106. The polygonal shape of the peripheral portion 108 may, for example, enable the diaphragm 100 to be fitted to a polygonal mounting portion of the diaphragm holder 102.

The polygonal shaped peripheral portion 108 can indicate that the diaphragm 100 is correctly mounted to the diaphragm holder 102, e.g. due to the complementary fit of the polygonal shaped peripheral portion 108 and the polygonal mounting portion of the diaphragm holder 102 to each other. The polygonal shaped peripheral portion 108 in combination with the polygonal mounting portion of the diaphragm holder 102 may also assist to ensure secure, non-rotatable mounting of the diaphragm 100 in the diaphragm holder 102.

The polygonal shape of the peripheral portion 108 may be a regular polygonal shape, with sides of equal length.

Fitting of the peripheral portion 108 to the polygonal mounting portion of the diaphragm holder 102 can be implemented in any suitable manner. In some embodiments, the peripheral portion 108 and the polygonal mounting portion are fitted to each other by an interference fit.

For example, the polygonal mounting portion may be in the form of a polygonal protrusion that protrudes from a base of the diaphragm holder 102, with the peripheral portion 108 of the diaphragm 100 being shaped and dimensioned to intimately wrap around surface(s) of the polygonal protrusion so as to be secured to the polygonal protrusion.

Referring to FIG. 2B, the polygonal shape of the peripheral portion 108 of the diaphragm 100 in the non-limiting example provided in the Figures is a square, e.g. rounded square, shape. Other polygonal shapes can be contemplated, such as triangular, e.g. rounded triangular, rectangular/oblong, e.g. rounded rectangular/oblong, pentagonal, e.g. rounded pentagonal, hexagonal, e.g. rounded hexagonal, heptagonal, e.g. rounded heptagonal, octagonal, e.g. rounded octagonal, nonagonal, e.g. rounded nonagonal, and decagonal, e.g. rounded decagonal.

It is noted that the term "rounded" in the context of the polygonal shape may refer to rounding of the vertices of the polygonal shape.

Irrespective of the precise number of sides of the polygonal shape of the peripheral portion 108, the diaphragm 100 may have a three-dimensional shape having an axis of rotational symmetry A1 that extends through a center of the polygonal shape to enable the diaphragm 100 to be fitted to the polygonal mounting portion of the diaphragm holder 102 in a plurality of angular orientations adoptable by rotating the diaphragm 100 about the axis A1.

The center of the polygonal shape can be regarded as being defined by the axis of rotational symmetry A1, with rotation about the axis A1 providing the plurality of (discrete) angular orientations.

This rotational symmetry of the diaphragm 100 can enhance user convenience, since the user is not burdened with having to locate, for example, a single orientation of the diaphragm 100 that enables the diaphragm 100 to be mounted to the diaphragm holder 102.

It is noted that the angular orientations may be regarded as being indistinguishable from each other in terms of functioning of the diaphragm 100 in the breast pump assembly.

In some embodiments, the three-dimensional shape of the diaphragm 100 has an order of rotational symmetry, about the axis of rotational symmetry A1, of between 2 and 10, preferably between 3 and 6, more preferably between 4 and 6. For example, in the case of the polygonal shape being a square or rounded square shape (see FIG. 2B), the order of rotational symmetry is 4. In such embodiments, the diaphragm 100 may be mounted to the diaphragm holder 102 in one of four angular orientations.

In other embodiments, in which the polygonal shape is an oblong shape, e.g. rectangular, so as to have a length dimension longer than a width dimension, the order of rotational symmetry is 2. In such embodiments, the diaphragm 100 may be mounted to the diaphragm holder 102 in one of two angular orientations.

As best shown in FIGs. 1 and 2A, a recessed portion 110 of the second exterior surface 106 is defined in a central region of the diaphragm 100. Such a recessed portion 110 can mean that the diaphragm 100 helps to make the breast pump assembly, or at least the milk expression kit 10, more compact, e.g. so as to facilitate fitting of the breast pump assembly, or at least the milk expression kit 10, in the mother's bra.

When the diaphragm 100 and the diaphragm holder 102 are arranged so that the second exterior surface 106 faces the mother's breast, the recessed portion 110 may be aligned with a nipple-receiving space of the nipple-receiving member 52, in which nipple-receiving space the mother's nipple NP is at least partially receivable.

In such embodiments, the recessed portion 110 can enable the diaphragm 100 to be arranged closer to the breast, with less risk of the nipple NP contacting the diaphragm 100 due to the recessed region 110 of the second exterior surface 106 providing additional space between the nipple NP and the diaphragm 100. By arranging the diaphragm 100 closer to the breast, the overall thickness of the breast pump assembly, or at least the milk expression kit 10, can be reduced, so as to take up less room in the mother's bra.

The alignment between the nipple-receiving space and the recessed portion 110 can mean that the recessed portion 110 and the nipple-receiving space are opposite each other, for example so that a central axis A2 extending through a center of the nipple-receiving space intersects with the diaphragm 100 at the recessed portion 110.

It is noted that the recessed region 110 of the second exterior surface 106 may correspond to a protruding region 111 of the first exterior surface 104. For example, a profile of the first exterior surface 104 may complement a profile of the second exterior surface 106, such that recessed region(s) 110 of one of the first and second exterior surfaces 104, 106 correspond to protruding region(s) 111 of the other of the first and second exterior surfaces 106, 104.

In some embodiments, and referring to FIG. 2A, a depth D1 of the recessed portion 110 is at least one third of a width W1 of the recessed portion 110, with the depth D1 being measured along the axis of rotational symmetry A1 and the width W1 being measured across the recessed region 110 perpendicular to the axis of rotational symmetry A 1. This minimum depth D1 requirement reflects statistical data gathered concerning nipple NP length to width ratios. In particular, the depth D1 of the recessed portion 110 being at least one third of the width of W1 the recessed portion 110 may enable the breast pump assembly, or at least the milk expression kit 10, to accommodate a largest nipple length to width ratio.

In some embodiments, the recessed portion 110 has a circular shape in plan when the diaphragm 100 is viewed facing the second exterior surface 106. For example, the recessed portion 110 may be dome-shaped. Such a dome-shaped recessed portion 110 can assist to ensure that the diaphragm 100 is always able to return to its original shape during pumping, which can help to create a stronger vacuum force. The stronger vacuum force can, in turn, help to enhance efficiency of milk extraction.

Other shapes of the recessed portion 110 can also be contemplated, such as cylindrical.

In embodiments in which the recessed portion 110 has a circular shape in plan, the width W1 may be a diameter of the circular shape.

In embodiments in which more than one width W1 extends across the recessed region 110 perpendicular to the axis of rotational symmetry A1, the width W1 utilized for establishing whether the depth D1 of the recessed portion 110 is at least one third of the width W1 of the recessed portion 110 may be a maximum width W1 across the recessed region 110 measured between points on the second exterior surface 106 that are separated from each other by a maximum distance across the recessed region 110.

When the variable pressure generator, e.g. pump, is providing the variable pressure between the first exterior surface 104 of the diaphragm 100 and the diaphragm holder 102, at least a central part of the first exterior surface 104 of the diaphragm 100 (e.g. at least the protruding region 111 corresponding to the recessed region 110 of the second exterior surface 106) may move towards and away from the diaphragm holder 102. For example, the central part of the first exterior surface 104, e.g. the protruding region 111 thereof, may move towards and touch an opposing surface of the diaphragm holder 102 when the variable pressure is being provided.

In other embodiments, the diaphragm 100 may be configured so that a central part, e.g. the protruding region 111, of the first exterior surface 104 of the diaphragm 100 may not move towards the diaphragm holder 102 in response to the variable pressure being provided, but rather surrounding part(s) of the diaphragm 100 may move towards and away from the diaphragm holder 102 while the central part remains stationary relative to the diaphragm holder 102. This may mean that even less space is used, so that the breast pump assembly, or at least the milk expression kit 10, can be made more compact.

As best shown in FIG. 2B, the diaphragm 100 comprises an intermediate portion 112A, 112B, 112C, 112D located between the recessed portion 110 and at least vertex regions 114A, 114B, 114C, 114D of the peripheral portion's 108 polygonal shape. The intermediate portion 112A, 112B, 112C, 112D of the diaphragm 100 can assist to increase the surface area of the diaphragm 100, in other words can provide an additional surface of the diaphragm 100 that can be moved, e.g. stretched, for vacuum generation.

In the non-limiting example shown in FIGs. 2A to 2C, the intermediate portion 112A, 112B, 112C, 112D comprises four corner portions of the square shaped, e.g. rounded square shaped, diaphragm 100, which corner portions are disposed between the recessed portion 110/protruding portion 111 and the vertex regions 114A, 114B, 114C, 114D of the peripheral portion's 108 polygonal shape.

The intermediate portion 112A, 112B, 112C, 112D of the diaphragm 100 can include, e.g. in addition to the corner portions, edge portions located between the recessed portion 1 10/protruding portion 111 and sides of the peripheral portion's 108 polygonal shape.

In some embodiments, such as shown in FIGs. 2A to 2C, ridge(s) and/or groove(s) 116 is/are defined in at least the second exterior surface 106 of the diaphragm 100. Such ridge(s) and/or groove(s) 116 can help to provide smooth milk flow off the second exterior surface 106 of the diaphragm 100, thereby ensuring efficient milk collection.

In some embodiments, a groove 116, e.g. an annular groove 116, defined in the second exterior surface 106 corresponds to a ridge 118, e.g. an annular ridge 118, of the first exterior surface 104. Alternatively or additionally, a ridge, e.g. an annular ridge, of the second exterior surface 106 corresponds to a groove, e.g. an annular groove, defined in the first exterior surface 104.

The ridge(s) and/or groove(s) 116 may extend at least partly, e.g. entirely, around the recessed region 110. For example, the ridge(s) and/or groove(s) 116 may annularly extend around the recessed region 110, e.g. the dome-shaped recessed region 110.

The ridge(s) and/or groove(s) 116 extending, e.g. annularly extending, around the recessed region 110 can help to increase the distance to which the diaphragm 100 is able to extend, thereby assisting to create a relatively high vacuum force.

As an alternative or in addition to the ridge(s) and/or groove(s) 116 extending at least partly around the recessed region 110, the ridge(s) and/or groove(s) may be defined on/in the recessed region 110 itself and/or on/in the protruding region 111 of the first exterior surface 104 that corresponds to the recessed region 110 of the second exterior surface 106.

In some embodiments, and referring again to FIG. 1, the diaphragm 100 is arranged in the milk expression kit 10 such that at least part of the second exterior surface 106 is, in use, angled to guide milk thereon towards the milk collection container 54. Thus, the orientation of at least part of the second exterior surface 106 of the diaphragm 100 can assist transfer of milk towards the milk collection container 54.

In some embodiments, such as shown in FIG. 1, the diaphragm 100 as a whole may be angled so that an overall orientation of the second exterior surface 106, in use, guides milk thereon towards the milk collection container 54.

The diaphragm 100 may be tilted, when the milk expression kit is assembled, such that the axis of rotational symmetry A1 extends at an oblique angle relative to the central axis A2 that extends through the center of the nipple-receiving space towards the diaphragm 100. This tilting of the diaphragm 100 may cause milk to be guided on the second exterior surface 106 towards the milk collection container 54.

It is noted at this point that the above-mentioned intersection of the central axis A2 extending through the center of the nipple-receiving space with the diaphragm 100 at the recessed portion 110 need not require that the center of the diaphragm 100 is intersected by the central axis A2. For example, in embodiments in which the diaphragm 100 is angled so that an overall orientation of the second exterior surface 106, in use, guides milk thereon towards the milk collection container 54, the central axis A2 extending through the center of the nipple-receiving space may intersect the diaphragm 100 at an off-center position due, at least in part, to the tilted orientation of the diaphragm 100. An example of this is illustrated in FIG. 1.

In other embodiments, a part of the second exterior surface 106 (rather than an overall orientation of the second exterior surface 106) is angled relative to neighboring part(s) of the second exterior surface 106, so that the part of the second exterior surface 106 guides milk thereon towards the milk collection container 54.

The present disclosure generally relates, at least in part, to the breast pump diaphragm 100 as such, since the diaphragm 100 can be supplied to the mother by itself, with the other components of the milk expression kit 10 or the breast pump assembly being already in the mother's possession, or sourced separately from the diaphragm 100.

Alternatively, the diaphragm 100 (or a plurality of such diaphragms 100) may be included in the milk expression kit 10 or in the breast pump assembly. In such embodiments, the mother may be conveniently supplied with the diaphragm(s) 100 along with other component(s) of the milk expression kit 10 (such as the diaphragm holder 102 and the nipple-receiving member 52) or, as the case may be, with other component(s) of the breast pump assembly (such as the variable pressure generator).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A milk expression kit (10) comprising:
a nipple-receiving member (52) in which a mother's nipple (NP) is at least partially receivable;
a diaphragm holder (102);
a breast pump diaphragm (100) for mounting to the diaphragm holder, an extremity (53) of the nipple-receiving member being located at a position inside the milk expression kit proximal to the diaphragm, with a diaphragm movement region being defined between the extremity and one of (i) the diaphragm and (ii) the diaphragm holder (102), when the milk expression kit is assembled for use, the diaphragm comprising:
a first exterior surface (104), a pump chamber being defined between the diaphragm holder and the first exterior surface when the milk expression kit is assembled for use;
a second exterior surface (106) facing away from the first exterior surface, the diaphragm being configured to transfer a variable pressure provided in the pump chamber to a breast chamber that is at least partly delimited by the second exterior surface; wherein a recessed portion (110) of the second exterior surface is defined in a central region of the diaphragm;
a peripheral portion (108) having a polygonal shape in plan when the diaphragm is viewed facing the first exterior surface or the second exterior surface; and
an intermediate portion (112A, 112B, 112C, 112D) located between the recessed portion and at least vertex regions (114A, 114B, 114C, 114D) of the peripheral portion's polygonal shape.

2. The milk expression kit (10) according to claim 1, wherein the diaphragm holder (102) comprises a polygonal mounting portion, the peripheral portion's (108) polygonal shape being for enabling the diaphragm (100) to be fitted to the polygonal mounting portion of the diaphragm holder.

3. The milk expression kit (10) according to claim 2, wherein the diaphragm (100) has a three-dimensional shape having an axis of rotational symmetry (A1) that extends through a center of the polygonal shape to enable the diaphragm to be fitted to the polygonal mounting portion in a plurality of angular orientations adoptable by rotating the diaphragm about the axis.

4. The milk expression kit (10) according to claim 3, wherein the three-dimensional shape of the diaphragm (100) has an order of rotational symmetry, about the axis of rotational symmetry (A1), of between 2 and 10, preferably between 3 and 6, more preferably between 4 and 6.

5. The milk expression kit (10) according to any one of claims 1 to 4, wherein a depth (D1) of the recessed portion (110) is at least one third of a width (W1) of the recessed portion, the depth being measured along an axis extending normal to a most recessed point of the second exterior surface (106) and the width being measured across the recessed region perpendicular to said axis extending normal to the most recessed point of the second exterior surface.

6. The milk expression kit (10) according to any one of claims 1 to 5, wherein the recessed portion (110) has a circular shape in plan when the diaphragm (100) is viewed facing the second exterior surface (106).

7. The milk expression kit (10) according to any one of claims 1 to 6, wherein ridge(s) and/or groove(s) (116) is/are defined in at least the second exterior surface (106) of the diaphragm (100).

8. The milk expression kit (10) according to claim 7, wherein the ridge(s) and/or groove(s) (116) extend(s) at least partly around the recessed region (110).

9. The milk expression kit (10) according to any one of claims 1 to 8, wherein the diaphragm (100) comprises an elastomeric material.

10. The milk expression kit (10) according to any one of claims 1 to 9, wherein positioning of the nipple-receiving member (52) relative to the diaphragm (100), in the milk expression kit assembled for use, is such that a nipple-receiving space inside the nipple-receiving member is aligned with the recessed portion (110) of the second exterior surface (106).

11. The milk expression kit (10) according to claim 10, wherein the alignment between the nipple-receiving space and the recessed portion (110) is such that a central axis (A2) extending through a center of the nipple-receiving space intersects with the diaphragm (100) at the recessed portion.

12. The milk expression kit (10) according to any one of claims 1 to 11, comprising a milk collection container (54), wherein the diaphragm (100) is arranged in the milk expression kit such that at least part of the second exterior surface (106) is, in use, angled to guide milk thereon towards the milk collection container.

13. The milk expression kit (10) according to any one of claims 1 to 12, wherein the milk expression kit is configured to be worn inside the mother's bra, such that at least the diaphragm holder (102), the diaphragm (100), and the nipple-receiving member (52) of the milk expression kit are received in the mother's bra when the milk expression kit is being worn by the mother.

14. A breast pump assembly comprising:
a variable pressure generator; and
the milk expression kit (10) according to any one of claims 1 to 13, wherein the variable pressure generator is configured to provide the variable pressure in the pump chamber defined between the first exterior surface (104) of the diaphragm (100) and the diaphragm holder (102).

15. The breast pump assembly according to claim 14, wherein the variable pressure generator is wearable and configured to generate the variable pressure while being worn; optionally wherein the variable pressure generator is wearable inside the mother's bra.
